(19) **Europäisches Patentamt / European Patent Office / Office européen des brevets**

(11) **EP 3 014 278 B1**

(12) # FASCICULE DE BREVET EUROPEEN

(45) Date de publication et mention de la délivrance du brevet:
**19.04.2017 Bulletin 2017/16**

(21) Numéro de dépôt: **14750536.6**

(22) Date de dépôt: **24.06.2014**

(51) Int Cl.:
**G01N 33/68** [(2006.01)]     **H01J 49/00** [(2006.01)]

(86) Numéro de dépôt international:
**PCT/FR2014/051576**

(87) Numéro de publication internationale:
**WO 2014/207367 (31.12.2014 Gazette 2014/53)**

(54) **PROCÉDÉ DE DÉTECTION ET DE QUANTIFICATION PAR SPECTROMÉTRIE DE MASSE ET PAR ACTIVATION D'ESPÈCES MOLÉCULAIRES IONISÉES**

VERFAHREN ZUM NACHWEIS UND ZUR QUANTIFIZIERUNG MITTELS MASSENSPEKTROMETRIE UND MITTELS AKTIVIERUNG IONISIERTER MOLEKULARER SPEZIES

METHOD OF DETECTION AND QUANTIFICATION BY MASS SPECTROMETRY AND BY ACTIVATING IONISED MOLECULAR SPECIES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.06.2013 FR 1356015**

(43) Date de publication de la demande:
**04.05.2016 Bulletin 2016/18**

(73) Titulaire: **Institut National de la Recherche Agronomique (INRA) 75007 Paris (FR)**

(72) Inventeur: **GIULIANI, Alexandre F-75014 Paris (FR)**

(74) Mandataire: **Jacobacci Coralis Harle 14-16, rue Ballu 75009 Paris (FR)**

(56) Documents cités:
**WO-A1-2013/021124**

- **KALLI A ET AL: "Electron capture dissociation of highly charged proteolytic peptides from Lys N, Lys C and Glu C digestion", MOLECULAR BIOSYSTEMS, vol. 6, no. 9, janvier 2010 (2010-01), page 1668, XP055111239, ISSN: 1742-206X, DOI: 10.1039/c003834b**

- **ANTOINE R ET AL: "Visible and ultraviolet spectroscopy of gas phase protein ions", PHYSICAL CHEMISTRY CHEMICAL PHYSICS, vol. 13, no. 37, janvier 2011 (2011-01), page 16494, XP055110898, ISSN: 1463-9076, DOI: 10.1039/c1cp21531k cité dans la demande**

- **BRUNET C ET AL: "Formation and fragmentation of radical peptide anions: Insights from vacuum ultra violet spectroscopy", JOURNAL OF THE AMERICAN SOCIETY FOR MASS SPECTROMETRY, vol. 23, no. 2, 15 novembre 2011 (2011-11-15), pages 274-281, XP055111086, ISSN: 1044-0305, DOI: 10.1007/s13361-011-0285-7**

- **GIULIANI A ET AL: "Structure and charge-state dependence of the gas-phase ionization energy of proteins", ANGEWANDTE CHEMIE INTERNATIONAL EDITION, vol. 51, no. 38, 21 août 2012 (2012-08-21), pages 9552-9556, XP055110956, ISSN: 1433-7851, DOI: 10.1002/anie.201204435 cité dans la demande**

- **ZUBAREV R A ET AL: "Multiple soft ionization of gas-phase proteins and swift backbone dissociation in collisions with <=99eV electrons", ANGEWANDTE CHEMIE INTERNATIONAL EDITION, vol. 49, no. 8, 20 janvier 2010 (2010-01-20), pages 1439-1441, XP055110962, ISSN: 1433-7851, DOI: 10.1002/anie.200905977 cité dans la demande**

Printed by Jouve, 75001 PARIS (FR)

**Description**

La présente invention se rapporte au domaine de la spectrométrie de masse. Plus précisément l'invention concerne un procédé de mesure quantitatif d'espèces moléculaires ionisées par spectrométrie de masse.

La spectrométrie de masse est une technique d'analyse de composés notamment en mélange, qui permet la détection, l'identification et, sous certaines conditions, la quantification d'espèces moléculaires ionisées même en très faible quantité.

Un spectromètre de masse classique comprend une source d'ionisation, un ou plusieurs analyseurs de masse et un système de détection d'ions. La source d'ionisation permet de générer des espèces ionisées en phase gazeuse à partir d'un échantillon à analyser. L'analyseur de masse permet de séparer les espèces ionisées en fonction de leur rapport masse sur charge (m/z) où m représente la masse de l'espèce ionisée et z son nombre de charge électrique. Le système de détection d'ions reçoit les espèces ionisées ainsi séparées par l'analyseur de masse. Un spectre de masse est obtenu par l'enregistrement de l'abondance des ions en fonction de leur rapport masse sur charge.

Dans le cas où la séparation des ions en fonction de leur rapport m/z est effectuée une seule fois, on parle spectre de masse simple ou MS. On peut aussi réaliser des séparations successives des ions à travers plusieurs étapes de spectrométrie de masse tandem, appelé $MS^n$, où n représente le niveau de séparation. Dans ce mode de fonctionnement, les espèces ionisées provenant de la source d'ionisation sont soumises à une première étape de séparation des ions dans laquelle un ou plusieurs ions d'intérêt sont sélectionnés. Cette sélection est effectuée par un analyseur de masse ou un dispositif permettant de sélectionner un domaine particulier de rapport m/z. Le ou les ions sélectionnés sont alors soumis à une étape d'activation. De manière non exclusive, cette activation se fait classiquement par des collisions avec un gaz inerte (azote, argon...), appelé CID pour *collision induced dissociation* et/ou par photodissociation (voir par exemple WO2012/066261_A1). L'activation des espèces ioniques d'intérêt, appelés précurseurs, peut conduire à leur fragmentation et générer des ions fragments dont le rapport m/z est différent de celui des précurseurs, la masse de l'ion fragment étant plus faible que celle de l'ion précurseur. L'étape de sélection et de fragmentation peut être répétée n-fois, soit spatialement dans autant d'analyseurs en masse en série, soit temporellement dans un piège à ion.

En mode MS simple, le rapport m/z est caractéristique de l'espèce d'intérêt. Dans le mode $MS^2$ ou MS/MS, on ajoute au rapport m/z de l'espèce d'intérêt un autre critère qui est le spectre de masse des ions fragments produits par l'activation du précurseur (voir par exemple WO2006/064132_A1). Dans les modes de spectrométrie de masse tandem $MS^n$, l'ensemble des spectres de masse obtenus à tous les n-niveaux de fragmentations, sont pris en compte dans l'analyse du précurseur.

Le dosage d'une espèce d'intérêt par spectrométrie peut se faire en mode MS simple de la façon suivante :

- le signal reçu par le détecteur d'ion est proportionnel à la quantité d'ions dans l'analyseur en masse
- la quantité d'ion issue de l'analyseur de masse est proportionnelle à la quantité d'ion-produit par la source d'ion
- la quantité d'ion-produit par la source d'ion est proportionnelle à la quantité d'échantillon présent dans la solution

Ainsi, on peut relier l'abondance des ions sur un spectre de masse simple à la quantité d'espèce d'intérêt présent dans l'échantillon. Dans le cas où on procède à une séparation chromatographique en amont de la source d'ion, l'intégration du pic chromatographique correspondant à l'espèce d'intérêt est alors reliée à la quantité de cette espèce dans l'échantillon. Néanmoins, le dosage en MS simple présente des inconvénients. Notamment la présence d'ions interférents de même rapport m/z ou de rapport m/z très proche de celui de l'espèce d'intérêt peut perturber ou même empêcher la mesure. En effet, le rapport m/z n'est pas un critère assez sélectif en soi.

Des méthodes basées sur la spectrométrie de masse tandem ont été développées pour pallier à ce manque de sélectivité. Le mode SRM pour *single reaction monitoring* repose sur l'utilisation de la MS/MS. Dans ce mode, le rapport masse sur charge de l'espèce d'intérêt est sélectionné dans une première étape de spectrométrie de masse tandem puis activé par exemple au moyen de collisions avec un gaz. Cette activation conduit à la fragmentation du précurseur et à l'apparition d'ions fragments qui sont spécifiques du précurseur et qui présentent un rapport m1/z1 différent du rapport m/z du précurseur. On appelle transition une voie de fragmentation particulière du précurseur vers un fragment spécifique : $m/z \rightarrow m1/z1$. Dans le mode SRM, seul le signal d'une fragmentation spécifique est suivi. Ainsi, en mode SRM, l'abondance d'un ion fragment spécifique est proportionnelle à la quantité de l'espèce d'intérêt présent initialement dans l'échantillon. Le mode SRM augmente fortement la spécificité de la mesure en réduisant très fortement la contribution d'espèces parasites. La spécificité de la mesure peut être encore augmentée en suivant plusieurs transitions. On parle alors de mode MRM pour *multiple reaction monitoring.* Néanmoins, dans le cas de matrices très complexes, il peut arriver que des composés de rapport m/z similaire à celui de l'espèce d'intérêt produisent des transitions de rapport masse sur charge suffisamment proche de celle que l'on suit pour perturber la mesure. Ce cas se rencontre notamment dans le cas du dosage des protéines. En effet, l'analyse des protéines par SRM ou MRM, implique une étape de digestion par protéolyse de l'échantillon contenant la protéine que l'on souhaite doser, car les protéines entières ne se prêtent pas l'analyse SRM. En effet, la fragmentation d'ions moléculaires de grande taille est très difficile. Cette digestion produit des peptides pour lesquels des transitions particulières seront suivies en analyse SRM ou MRM. Généralement, les peptides produisent des fragmentations plus complexes que celles des petites molécules (métabolites, drogues...) ce qui complique le choix des transitions SRM. De plus, la présence d'autres protéines dans l'échantillon peut conduire à l'apparition de peptides qui produiront des transitions identiques ou très proches de celles que l'on suit et qui perturberont la mesure [voir P. Picotti and R. Aebersold, 2012, « Selected reaction monitoring-based proteomics : workflows, potential, pitfalls and future directions », Nature Methods, 9 555-66]. La publication « Electron capture dissociation of highly charged proteolytic peptides from Lys N, Lys C and Glu C digestion" A. Kalli and K. Hakansson, Mol. Biosyst., 2010, 6, 1668-1681 décrit l'application d'une technique de fragmentation par capture d'électrons (ECD) appliquée à des ions peptides issus de la digestion de protéines, qui conduit à la scission des ions peptides en de nombreux fragments ou résidus de peptides.

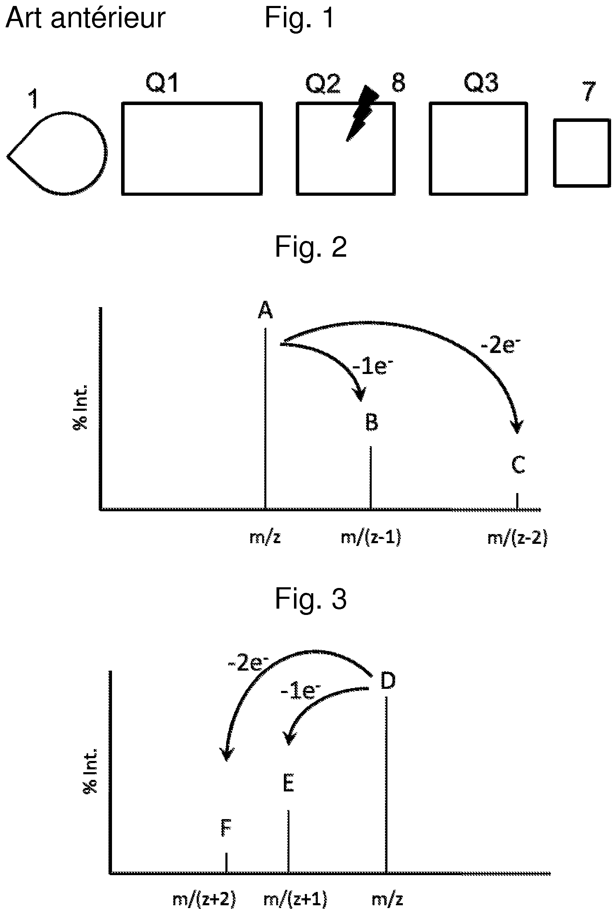

La figure 1 représente schématiquement un système de spectromètre de masse de type triple quadrupole comme exemple d'appareil pour l'analyse par spectrométrie de masse tandem. Le système de la figure 1 comprend une source d'ionisation 1, un premier analyseur de masse Q1, un second analyseur de masse Q2 couplé à un moyen d'activation des ions 8, un autre analyseur de masse Q3 et un système de détection d'ions 7. La méthode d'activation d'ion la plus couramment utilisée est appelée fragmentation induite par collision ou CID pour Collision Induced Dissociation. La CID consiste à fragmenter les ions par collision inélastique entre les ions et des espèces neutres, telles que les atomes ou molécules d'un gaz rare (hélium, azote, argon...). Cependant, la CID produit une fragmentation non sélective des ions : tous les ions présents dans le piège à ions peuvent être fragmentés par collision avec le gaz rare de collision. En mode SRM ou MRM, l'analyseur Q1 de la figure 1 laisse passer les ions correspondants aux espèces d'intérêt en fonction de leur rapport m/z. L'analyseur Q2 reçoit ces ions et les fragmente en ion-produits. Les ions fragments ainsi générés sont transmis dans l'analyseur Q3 où ils sont filtrés et sélectionnés en fonction de leur rapport m/z spécifique. L'abondance des ions fragments qui passent à travers Q3 est alors enregistrée par le détecteur. Ce type d'analyse peut aussi se faire en utilisant d'autres configurations instrumentales (analyseurs hybrides, pièges à ions...).

Récemment une méthode est apparue dans laquelle l'activation est basée sur l'absorption d'une onde électromagnétique provenant d'un laser par des ions stockés dans un piège [Lemoine J, Dugourd P, Salvador A, Antoine R and Bretonniere Y (2010) Brevet FR 2967 780]. Cette activation par absorption d'un rayonnement laser (appelée LID pour *Laser Induced Dissociation*) permet, en principe, une activation sélective en fonction de la longueur d'onde d'émission du laser. En effet, la longueur d'onde du laser peut être choisie de façon à ce que seulement l'espèce d'intérêt absorbe le rayonnement électromagnétique. Ainsi, les fragmentations produites par LID sont spécifiques de l'espèce d'intérêt. Les composés présents dans le mélange ne peuvent plus interférer dans l'analyse SRM (ou MRM) car ils n'absorbent pas le rayonnement électromagnétique spécifique. La fragmentation induite par laser est rendue sélective par les caractéristiques de l'absorption du rayonnement.

Un inconvénient majeur du dosage par SRM (ou MRM) est qu'il implique une ou plusieurs fragmentations des ions d'intérêt en fragments de plus petite taille. Les techniques d'analyse par spectrométrie de masse par Single Reaction Monitoring (SRM) et Multiple Reaction Monitoring (MRM) ne sont pas applicables si les espèces d'intérêt ne peuvent pas être fragmentées. Il est difficile voire impossible de fragmenter certaines espèces moléculaires, notamment en CID. A titre d'exemple non restrictif, les bio-polymères en général et les protéines en particulier ne peuvent être soumises intactes et entières à une analyse de ce type. En effet, la fragmentation des protéines ou des biopolymères entiers est très difficile et donne peu de fragments lorsque leur taille augmente. Cela explique pourquoi une étape de digestion des protéines est mise en place avant l'analyse par spectrométrie de masse.

Il existe donc un besoin d'un procédé de mesure quantitative et sélective par spectrométrie de masse.

Un des buts de l'invention est de proposer un procédé de spectrométrie de masse permettant de dépasser les limitations des techniques de SRM et MRM, permettant notamment l'analyse directe et sélective des espèces moléculaires et des (bio)polymères intacts, en particulier d'espèces moléculaires difficiles ou impossible à fragmenter.

La présente invention a pour but de remédier aux inconvénients précités de l'état de la technique et concerne plus particulièrement un procédé de mesure par spectrométrie de masse d'espèces moléculaires ionisées dans un mélange complexe.

Selon l'invention, le procédé comprend les étapes suivantes :

a. ionisation d'un analyte en mélange complexe pour former un ion précurseur d'une espèce ionisée d'intérêt et au moins un ion interférent provenant du mélange complexe, l'ion précurseur de ladite espèce ionisée d'intérêt et ledit au moins un ion interférent ayant un même ratio masse sur charge *m/z,* où m représente la masse et z le nombre de charge électrique de l'ion précurseur de ladite espèce ionisée d'intérêt ;

b. activation de l'ion précurseur de ladite espèce ionisée d'intérêt et du au moins un ion interférent de même ratio masse sur charge, par interaction avec un faisceau d'espèces neutres, d'ions, d'électrons ou de photons ayant une énergie prédéterminée en fonction de l'énergie d'ionisation de l'ion précurseur de ladite espèce ionisée d'intérêt ou de l'énergie nécessaire au détachement d'électron de l'ion précurseur de ladite espèce ionisée d'intérêt, ladite

3

activation étant adaptée pour produire un ion-produit, par transition de charge sans fragmentation de l'ion précurseur de ladite espèce ionisée d'intérêt, l'ion-produit ayant la même masse m que l'ion précurseur de ladite espèce ionisée d'intérêt et une charge électrique z' telle que z' est un nombre entier non nul différent de z, z' étant de préférence choisi parmi *z+1, z+2, z+3, z-1, z-2, z-3*, selon la polarité de l'ion précurseur, de manière à ce que l'ion-produit de ladite espèce ionisée d'intérêt ait un ratio masse sur charge *m/z'* différent du ratio masse sur charge du ou des ions-produits issus par activation du au moins un ion interférent ;

c. séparation de l'ion-produit de l'espèce ionisée d'intérêt ayant ledit ratio masse sur charge *m/z'* prédéterminé spécifique de l'espèce ionisée d'intérêt ;

d. détection sélective de l'ion-produit de l'espèce ionisée d'intérêt ayant ledit ratio masse sur charge *m/z'* prédéterminé spécifique de l'espèce ionisée d'intérêt.

Ainsi, le procédé de l'invention permet d'analyser par spectrométrie de masse et de détecter un ou plusieurs ions ayant subi une ou plusieurs transitions de charge sans nécessiter une fragmentation des ions précurseurs, donc sans modification de la masse de l'ion précurseur de l'espèce ionisée d'intérêt. Le procédé de l'invention permet d'analyser par spectrométrie de masse des espèces moléculaires ionisées dont la fragmentation est difficile voire impossible.

Selon un mode de réalisation particulier et avantageux, le procédé comporte en outre une étape supplémentaire e) suite à l'étape d), l'étape e) comprenant une mesure de l'abondance de l'ion-produit ayant ledit ratio masse sur charge *m/z'* prédéterminé spécifique de l'espèce ionisée d'intérêt.

Selon un mode de réalisation particulier et avantageux, ledit ion précurseur de l'espèce ionisée d'intérêt a une polarité positive et l'étape b) d'activation comprend une étape d'ionisation sans fragmentation par interaction avec un faisceau de photons, d'électrons ou d'ions de manière à former un ion-produit ayant la même masse *m* que ledit ion précurseur de l'espèce ionisée d'intérêt et une polarité positive telle que le nombre de charge électrique z' de l'ion-produit est de préférence égal à *z+1* par simple ionisation ou respectivement à *z+2* par double ionisation successive ou directe.

Selon un mode de réalisation particulier et avantageux, ledit ion précurseur de l'espèce ionisée d'intérêt a une polarité négative et l'étape b) d'activation comprend une étape de détachement d'électron(s) sans fragmentation par interaction avec un faisceau de photons ou d'électrons de manière à produire un ion-produit ayant la même masse m que ledit ion précurseur de l'espèce ionisée d'intérêt et une polarité négative telle que le nombre de charge électrique z'de l'ion-produit est de préférence égal à *z-1* par simple détachement d'électron ou respectivement à *z-2* par double détachement d'électron successif ou direct.

Selon un mode de réalisation particulier et avantageux, ledit ion précurseur de l'espèce ionisée d'intérêt a une polarité positive, avec z supérieur ou égal à 2, et l'étape b) d'activation comprend une étape de capture d'électron ou de transfert d'électron sans fragmentation par interaction avec un faisceau d'électron d'énergie appropriée de manière à produire un ion-produit ayant la même masse *m* que ledit ion précurseur de l'espèce ionisée d'intérêt et une polarité positive telle que le nombre de charge électrique z' de l'ion-produit est de préférence égal à *z-1* par simple capture ou transfert d'électron ou respectivement à *z-2* par double capture ou transfert d'électron.

Selon un mode de réalisation particulier et avantageux, ledit ion précurseur de l'espèce ionisée d'intérêt a une polarité positive et l'étape b) d'activation comprend une étape de transfert d'électron sans fragmentation par interaction avec un anion réactif d'affinité électronique appropriée de manière à former un ion-produit ayant la même masse *m* que ledit ion précurseur de l'espèce ionisée d'intérêt et une polarité positive telle que le nombre de charge électrique z' de l'ion-produit est de préférence égal à *z-1* par simple capture d'électron ou respectivement à *z-2* par double capture d'électron.

Selon un mode de réalisation particulier et avantageux, ledit ion précurseur de l'espèce ionisée d'intérêt a une polarité négative et l'étape b) d'activation comprend une étape de transfert d'électron(s) sans fragmentation par interaction avec un faisceau d'ion positif réactif de manière à produire un ion-produit ayant la même masse *m* que ledit ion précurseur de l'espèce ionisée d'intérêt et une polarité négative telle que le nombre de charge électrique z' de l'ion-produit est de préférence égal à *z-1* par simple transfert d'électron ou respectivement à *z-2* par double transfert d'électron.

Selon un mode de réalisation particulier et avantageux, le procédé de mesure comprend en outre les étapes suivantes :

- une étape a1) suite à l'étape a) d'ionisation, l'étape a1) comprenant la détection et l'enregistrement d'un spectre de masse simple de référence ;
- une étape c1) de détection et d'enregistrement d'un spectre de masse tandem relatif à un ion précurseur de référence de ratio (*m/z*)$_{réf}$ donnant au moins un ion-produit de ratio masse sur charge (*m/z'*) ; et
- une étape d1) de mesure de l'abondance de l'ion-produit de l'espèce ionisée d'intérêt.

Selon des aspects particuliers du procédé de mesure de l'invention :

- l'étape b) d'activation est réalisée par interaction d'un ion précurseur avec un faisceau de photons issu d'un laser, d'une lampe à décharge ou d'une source de rayonnement synchrotron ou d'un faisceau d'électrons ;
- l'étape b) d'activation est réalisée par interaction d'un ion précurseur avec un faisceau de photons ayant une longueur d'onde comprise dans le domaine UV ou VUV, de préférence comprise entre 3eV et 20 eV ou entre 3 eV et 40 eV ;
- l'étape b) d'activation est réalisée par interaction d'un ion précurseur avec un faisceau d'électrons ayant une énergie cinétique comprise entre 0 et 100 eV;
- l'étape b) d'activation est réalisée par interaction d'un ion précurseur avec un faisceau d'électrons issu d'un canon à électrons (filament, cathode...) ; dans le cas où l'on cherche à produire une capture d'électron, l'énergie du faisceau d'électrons est relativement faible (<1 eV) et dans le cas où l'on cherche l'ionisation ou le détachement d'électron, l'énergie des électrons est plus importante, typiquement de 10 eV jusqu'à 100 eV, ou jusqu'à 10 keV.
- l'étape b) d'activation est réalisée par interaction d'un ion précurseur avec un faisceau de cations d'énergie supérieure au keV ;
- le procédé comprend en option une étape préalable de fragmentation avant l'étape b) d'ionisation, de détachement d'électron, de transfert ou de capture d'électron(s) sans fragmentation ;
- les étapes b) d'activation et c) de séparation d'un ion-produit sont réalisées dans un piège à ions.

Ces valeurs d'énergie de photons, d'ions ou d'électrons sont ici indiquées à titre d'exemple et nullement limitatives.

De façon avantageuse, l'énergie du faisceau d'activation est adaptée en fonction des propriétés physico-chimiques (énergie d'ionisation, énergie de liaison de l'électron, affinité électronique) de l'ion précurseur de l'espèce ionisée d'intérêt, en vue de produire un ion-produit particulier, spécifique de l'espèce ionisée d'intérêt.

Pour mettre en oeuvre l'invention un dispositif de spectrométrie de masse comprend une source d'ionisation adaptée pour former un ion précurseur d'une espèce ionisée d'intérêt à partir d'un mélange complexe, l'ionisation dudit mélange complexe produisant ledit ion précurseur de ladite espèce ionisée d'intérêt et au moins une autre espèce ionisée interférente ayant un même ratio masse sur charge $m/z$ prédéterminé, où $m$ représente la masse et $z$ le nombre de charge électrique de l'ion précurseur de l'espèce ionisée d'intérêt. En outre le dispositif de spectrométrie de masse comprend :

- des moyens de couplage entre une cellule d'activation ou une région d'un spectromètre de masse adaptée pour recevoir, d'une part, ledit ion précurseur de l'espèce ionisée d'intérêt et ladite au moins une autre espèce ionisée interférente de même ratio masse sur charge provenant de l'ionisation du mélange complexe et, d'autre part, un faisceau d'espèces neutres, de photons, d'électrons ou d'ions, ledit faisceau ayant une énergie et une puissance d'activation prédéterminées en fonction des propriétés physico-chimiques de l'ion précurseur, ledit faisceau étant adapté pour former, par transition de charge électrique et sans fragmentation de l'ion précurseur de ladite espèce ionisée d'intérêt, un ion-produit ayant la même masse $m$ que l'ion précurseur de ladite espèce ionisée d'intérêt et un nombre de charge électrique $z'$ tel que $z'$ est un nombre entier non nul différent de $z$, $z'$ étant de préférence choisi parmi $z+1$, $z+2$, $z+3$, $z-1$, $z-2$, $z-3$ de manière à ce que l'ion-produit de ladite espèce ionisée d'intérêt ait un autre ratio masse sur charge $m/z'$, ledit autre ratio masse sur charge $m/z'$ étant différent du ratio masse sur charge d'un autre ion-produit issu de ladite au moins une autre espèce ionisée interférente ;
- un analyseur de masse adapté pour séparer ledit ion-produit ayant ledit autre ratio masse sur charge ($m/z'$) prédéterminé spécifique de l'espèce ionisée d'intérêt ; et
- un système de détection adapté pour détecter sélectivement ledit ion-produit ayant ledit autre ratio masse sur charge ($m/z'$) prédéterminé spécifique de l'espèce ionisée d'intérêt.

Selon un aspect particulier du dispositif de spectrométrie de masse le système de détection est configuré pour mesurer la quantité d'ion-produit ayant subi une ionisation supplémentaire sans fragmentation ou un détachement d'électron(s) supplémentaire sans fragmentation.

Le dispositif de spectrométrie permet de mesurer l'abondance d'au moins un ion-produit ayant subi une modification de charge électrique (par ionisation, détachement d'électron, capture d'électron ou transfert d'électron) sans fragmentation en fonction de l'évolution de l'abondance de l'ion précurseur cible.

L'invention concerne aussi l'application du procédé de mesure par spectrométrie de masse d'espèces moléculaires en mélange complexe selon l'un des modes de réalisation décrits au dosage de protéines, de bio-polymères, de polymères, de macromolécules, de virus, de nanoparticules, de micro-organismes d'assemblages supramoléculaires ou d'assemblages de bio-polymères.

De façon particulière et avantageuse, le système de détection est configuré pour mesurer la quantité d'ion-produit ayant subi une ionisation supplémentaire sans fragmentation ou un détachement d'électron(s) supplémentaire sans fragmentation.

L'invention trouvera une application particulièrement avantageuse dans la spectrométrie de masse appliquée à des molécules complexes, aux macromolécules, et assemblages supramoléculaires, aux virus, aux nanoparticules ou à des micro-organismes.

L'invention permet avantageusement d'identifier et de quantifier des espèces moléculaires par spectrométrie de masse sans nécessiter de fragmenter les espèces moléculaires cibles.

Cette description donnée à titre d'exemple non limitatif fera mieux comprendre comment l'invention peut être réalisée en référence aux dessins annexés sur lesquels :

- la figure 1 représente schématiquement un procédé d'analyse par spectrométrie de masse selon l'art antérieur ;
- la figure 2 représente schématiquement un exemple d'un spectre de masse tandem de détachement d'électron d'ion négatif ;
- la figure 3 représente schématiquement un exemple d'un spectre de masse tandem d'ionisation d'ion positif ;
- la figure 4 représente schématiquement un spectromètre de masse de type triple quadrupole selon un mode de réalisation de l'invention ;
- la figure 5 représente schématiquement un exemple de dispositif basé sur l'utilisation d'un piège à ion couplé à une source de rayonnement électromagnétique ;
- la figure 6 représente les chromatogrammes obtenus par un procédé classique de SRM pour quatre peptides issus de la digestion trypsique de la protéine de BSA (albumine de sérum bovin) ;
- la figure 7 représente les chromatogrammes obtenus par un procédé classique de SRM pour quatre peptides résultant de la digestion d'une protéine de BSA en mélange avec la digestion trypsique d'un extrait total de protéine de la bactérie E. Coli ;
- la figure 8 représente les chromatogrammes obtenus par analyse SRM avec transition de charge sans fragmentation, appliquée à un standard de BSA entière mélangé à un extrait total brut bactérien.

Le principe de l'invention est basé sur la méthode SRM ou MRM, mais ne repose pas sur la détection de fragmentations induites dans la ou les étapes de spectrométrie tandem mais plutôt sur l'observation d'ions moléculaires intacts mais qui ont subi une (ou plusieurs) ionisation(s), un (ou plusieurs) détachement d'électron(s), une (ou plusieurs) capture d'électron(s) ou un (ou plusieurs) transfert d'électron(s) suivant que l'ion précurseur soit positif ou négatif.

Ainsi, dans le cas d'un ion précurseur négatif, ou anion précurseur, de charge z supérieure ou égale à deux, l'activation de cet anion précurseur peut conduire au détachement d'électron(s) selon l'équation de détachement d'un électron :

$$M^{z-} \rightarrow M^{(z-1)-} + e^{-} \hspace{4cm} 1.$$

Cette activation, représentée par une flèche, peut se faire par impact d'électrons, par collision avec des neutres, par collision avec des ions ou par absorption de photon(s) provenant d'un laser, d'une lampe, d'un rayonnement synchrotron ou d'un laser à électron libre.

Dans ce cas, il est nécessaire que la charge de l'anion précurseur soit supérieure ou égale à deux afin de conserver au moins une charge négative sur l'ion-produit de la réaction pour permettre sa détection par spectrométrie de masse.

A titre illustratif, on a représenté sur la figure 2 un spectre de masse tandem suite, par exemple, au photodétachement d'un ou deux électrons d'un ion négatif. L'anion précurseur A présente un ratio masse sur charge $m/z$. Par absorption d'un rayonnement de photons ayant une énergie supérieure ou égale à l'énergie requise pour arracher un électron (10) de l'anion précurseur A, on obtient un nouvel anion-produit B qui a un électron de moins que l'anion précurseur A et présente donc un ratio masse sur charge égal à $m/(z-1)$, où z représente le nombre de charge électrique de l'anion précurseur A. Puisque l'anion-produit B a un électron de moins que l'anion précurseur A, la charge de l'anion-produit B est inférieure d'une unité à la charge de l'anion précurseur A. On peut aussi obtenir un nouvel anion-produit C qui a deux électrons de moins que l'anion précurseur A durant la même séquence d'activation. L'anion-produit C présente donc un ratio masse sur charge égal à $m/(z-2)$. L'anion-produit C peut être obtenu par arrachement simultané de deux électrons de l'anion précurseur A ou par arrachement d'un électron de l'anion-produit B.

Dans une variante, les anions-produits B et C peuvent être produits par transfert d'électron vers un cation dans un mécanisme appelé N-ETD (*Negative Electron Transfer Dissociation*).

Dans le cas d'un ion précurseur positif, ou cation précurseur, de charge z supérieure ou égale à un, l'activation par transition de charge peut conduire à l'ionisation de ce cation avec augmentation de sa charge selon l'équation d'ionisation :

$$M^{z+} \rightarrow M^{(z+1)+} + e \qquad\qquad 2.$$

De même, cette activation, représentée par une flèche, peut se faire par impact d'électrons, par collision avec des neutres, par collision avec des ions ou par absorption de photon(s) provenant d'un laser, d'une lampe, d'un rayonnement synchrotron ou d'un laser à électron libre.

De manière analogue à la figure 2, on a représenté sur la figure 3 un exemple d'un spectre de masse tandem de photoionisation d'ion positif. Le cation précurseur D présente un ratio masse sur charge *m/z*. Par absorption d'un rayonnement de photons ayant une énergie supérieure ou égale à l'énergie requise pour induire une ionisation (110) du cation précurseur D, on obtient un nouveau cation-produit E dont la perte d'un électron conduit à une charge positive supplémentaire par rapport au cation précurseur D et présente donc un ratio masse sur charge égal à *m/(z+1)*, où *z* représente le nombre de charge électrique du cation précurseur D. De manière analogue, par interaction avec un faisceau de photons ou d'électrons, on peut aussi former un nouveau cation-produit F qui a deux charges électroniques positives de plus que le cation précurseur D. Le cation-produit F présente donc un ratio masse sur charge égal à *m/(z+2)*. Le cation-produit F peut être obtenu par arrachement simultané de deux électrons du cation précurseur D ou par arrachement d'un électron du cation-produit E.

Dans une variante, le cation précurseur de rapport m/z de charge z supérieure à 2 peut aussi subir une capture d'électron (ECD) ou un transfert d'électron (ETD), respectivement par interaction avec un faisceau d'électron ou avec un faisceau d'anion, pour former un nouveau cation-produit de charge (z-1) et de rapport m/(z-1). Bien que se rapportant à des cations, cette variante ressemble au cas présenté à la figure 2.

On observe donc que la réaction de détachement d'électrons génère des anions produits B, C qui sont décalés dans la direction du ratio m/z croissant pour un anion précurseur. Au contraire, la réaction d'ionisation génère des cations produits E, F qui sont décalés dans la direction du ratio m/z décroissant pour un cation précurseur. La réaction d'activation par arrachement d'électron ou par ionisation des ions précurseurs induit une modification du spectre de masse différente selon la polarité des ions précurseurs et la méthode d'activation. De manière complémentaire, cela peut permettre de déterminer l'état de charge de l'ion précurseur ou ion cible.

L'activation par transition de charge électrique de l'ion précurseur, ou ion cible, peut être produite par impact d'électrons, par collision avec des neutres, par collision avec des ions ou par absorption de photon(s) provenant d'un laser, d'une lampe, d'un rayonnement synchrotron ou d'un laser à électron libre et dirigés sur l'ion précurseur.

Pour cela, après la source d'ionisation et après l'étape de sélection, on apporte à l'ion précurseur une énergie suffisante pour arracher un ou plusieurs électrons ou une énergie suffisante pour induire une ionisation supplémentaire de l'ion précurseur par capture d'un ou de plusieurs électrons.

Suivant les moyens d'activation et le temps d'activation mis en jeu, les réactions d'ionisation et/ou de détachement d'électron peuvent conduire à l'émission de plusieurs électrons soit directement, soit de façon séquentielle selon respectivement l'une des équations suivantes :

$$M^{z+} \rightarrow M^{(z+n)+} + ne^{-} \qquad\qquad 3.\text{Ionisation multiple}$$

$$M^{z-} \rightarrow M^{(z-n)-} + ne^{-} \qquad\qquad 4.\text{ Détachement multiple d'électron}$$

Les réactions 1 à 4 ont la particularité de donner un ion-produit dont le rapport masse sur charge est spécifique d'une masse et d'une charge déterminées de l'ion précurseur, donc d'un rapport masse sur charge précis, ce qui permet d'analyser de façon non ambiguë une espèce moléculaire particulière à l'origine de l'ion précurseur.

A titre d'exemple non restrictif, l'analyse par MS simple de polypeptides pourra être rendue difficile par la présence d'ions interférents dont le rapport m/z est identique ou très proche de ceux des espèces d'intérêts, mais dont la masse et la charge sont différents. L'application de la méthode proposée dans l'invention permet de lever ces ambiguïtés. En effet, l'analyse d'une transition de charge particulière selon l'une ou plusieurs des réactions 1 à 4 donne un ion-produit spécifique du rapport m/z du polypeptide et qui est différent de ceux des ions interférents de la matrice complexe.

A titre d'exemple non restrictif, le dosage SRM ou MRM d'une protéine d'intérêt dans un mélange complexe de protéines nécessite de passer par une étape de digestion enzymatique du milieu contenant toutes les protéines. En SRM (ou MRM) classique, une (ou plusieurs) transitions particulières sont suivies pour un ou plusieurs peptides issus de la digestion protéolytique de la protéine d'intérêt. Néanmoins, la digestion d'autres protéines présentes dans le milieu peut générer des peptides protéolytiques qui viennent interférer avec la mesure SRM ou MRM classique (soit parce que ces peptides sont identiques à ceux que l'on regarde ou bien parce qu'ils donnent des transitions identiques).

L'application de la méthode proposée ici n'est pas sensible à ces problèmes d'interférence, car elle est spécifique de la protéine d'intérêt. Ainsi, les protéines du milieu peuvent avoir des rapports m/z très proches, voire indiscernables de celui de l'ion précurseur d'intérêt. Cependant, le suivi d'une ou plusieurs transitions de charge selon les équations 1 à 4 donne un ou plusieurs ions produit(s) spécifique(s) du rapport m/z de l'espèce d'intérêt.

Cette méthode peut s'appliquer à titre d'exemple non restrictif au dosage d'autres biomolécules entières, de complexes et assemblages non-covalents, de polymères synthétiques, de nanoparticules, de virus ou d'autres micro-organismes, etc. En effet, certains ions précurseur ne peuvent pas être ou bien sont difficilement fragmentés par les méthodes classiques d'activation et ne peuvent donc pas faire l'objet d'une analyse SRM (ou MRM). Néanmoins, ces objets peuvent subir des transitions de charge, ce qui rend méthode proposée applicable et permet leur dosage.

En particulier, dans le cas d'un mélange complexe contenant l'ion d'intérêt et un ion interférent de même rapport masse sur charge (m/z), l'ionisation, le détachement d'électron, la capture ou le transfert d'électrons conduisent à des ion-produits spécifiques issus de la réaction sur l'ion précurseur d'intérêt, qui sont différents des ion-produits issus de réactions venant des ions interférents, ce qui permet de séparer les contributions de l'ion précurseur d'intérêt de celles des ions interférents. Ainsi, l'énergie d'activation ou d'excitation n'est pas nécessairement spécifique à une espèce d'intérêt et les contaminants peuvent aussi subir des transitions de charge lors de l'activation, cependant les rapports masse sur charge des ion-produits issus de ces réactions sont différents respectivement pour l'espèce d'intérêt et pour les interférents.

De plus, l'ionisation et le détachement d'électron ne peuvent pas être considérés comme des fragmentations, car la masse des ions précurseurs n'est pas affectée mais seulement leur charge. On note ($m/z$) le ratio masse sur charge de l'ion précurseur d'intérêt et ($m'/z'$) le ratio masse sur charge d'un ion interférent ou contaminant. Supposons que ($m/z$) est égal à ($m'/z'$) alors que la masse m de l'ion précurseur est différente de la masse m' de l'ion interférent et que le nombre de charge électrique $z$ de l'ion précurseur est différent du nombre de charge électrique $z'$ de l'ion interférent. La spectrométrie de masse classique ne permet pas de distinguer l'ion précurseur de l'ion interférent puisque ($m/z$) est égal à ou très proche de ($m'/z'$). Cependant, suite à une activation induisant une ionisation ou un détachement d'électron, ces deux espèces ioniques donnent des ion-produits différents.

Dans le cas d'ions positifs, ayant des charges $z$ et $z'$ différentes, on obtient :

$m/z \rightarrow m/(z+1)$ pour l'ion précurseur d'intérêt
$m'/z' \rightarrow m'/(z'+1)$ pour l'ion interférent

Ainsi, même si le contaminant possède un rapport masse sur charge indiscernable de celui de l'ion précurseur d'intérêt (m/z égal à ou très proche de m'/z'), après réaction (transition de charge) l'ion-produit présente un ratio masse sur charge m/(z+1) différent de m'/(z'+1).

De même, dans le cas d'anions :

$m/z \rightarrow m/(z-1)$
$m'/z' \rightarrow m'/(z'-1)$

où $m/(z-1)$ est différent de $m'/(z'-1)$.

Dans le cas de réactions d'ionisation multiple ou de détachement d'électrons multiples, les transitions produisent des espèces ioniques ayant un ratio masse sur charge : $m/(z+1)$, $m/(z+2)$ ou $m/(z+3)$ ... pour des ions positifs et respectivement des espèces ioniques ayant un ratio masse sur charge : $m/(z-1)$, $m/(z-2)$, $m/(z-3)$ ... pour des ions négatifs.

La détection des espèces ioniques de ratio $m/(z+1)$, $m/(z+2)$ ou $m/(z+3)$ pour des ions positifs, $m/(z-1)$, $m/(z-2)$, $m/(z-3)$ ... pour des ions négatifs peut servir dans le cadre d'une analyse de type MRM.

Le procédé de spectrométrie de masse tandem par transition de charge possède de nombreux avantages. L'ionisation, le détachement d'électron, la capture ou le transfert d'électrons peuvent se produire sur des espèces ionisées entières, voire même directement sur des assemblages non-covalents. Le procédé de l'invention permet de faire l'analyse de bio-polymères entiers (protéines, acides nucléiques...) sans passer par une étape de digestion contrairement aux techniques d'analyse basées sur la fragmentation. La méthode proposée permet donc l'analyse directe d'espèces moléculaires ionisées de toute taille.

De plus, cette méthode est applicable à des espèces qui ne peuvent pas être fragmentées, comme les nanoparticules, les virus ou tout autre type de cellules ou assemblages de bio-polymères ou de cellules du moment qu'ils peuvent être placés en phase gazeuse dans un spectromètre de masse.

La méthode de spectrométrie de masse n'est pas spécifique d'un moyen d'activation particulier. En effet, l'ionisation ou le détachement d'électron et peuvent avoir pour origine l'impact d'électrons, les collisions énergétiques avec des neutres, les collisions avec des ions ou l'absorption de photon(s) issu d'une source laser, d'une lampe, d'un rayonnement synchrotron, ou d'un laser à électron libre. La capture d'électron menant à une réduction de la charge peut aussi survenir suite à l'interaction avec un faisceau d'électrons. Le transfert d'électron menant à une modification de la charge peut aussi survenir suite à l'interaction avec un ion réactif.

Cette méthode peut être implémentée sur une variété d'instruments de spectrométrie de masse existants.

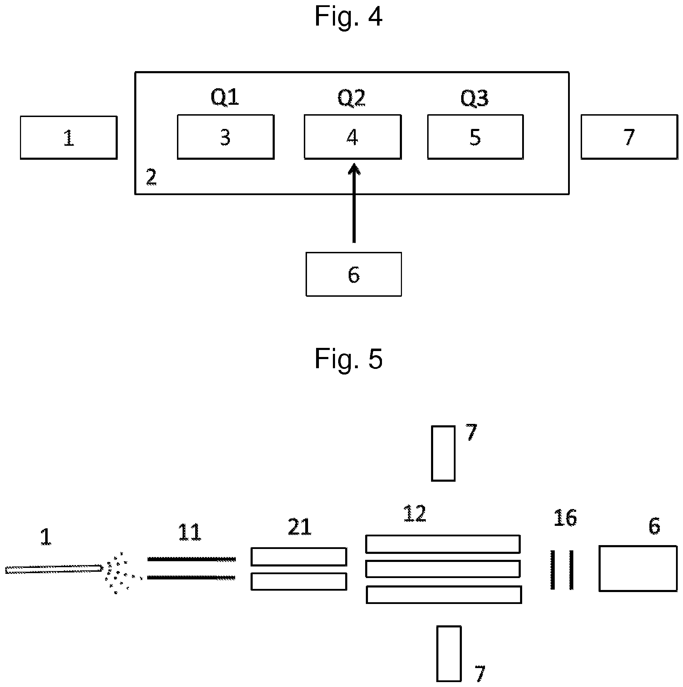

Sur la figure 4 on a représenté un exemple d'un système de spectrométrie de masse de type triple quadrupole selon un mode de réalisation de l'invention.

Le dispositif de spectrométrie de masse de la figure 4 comprend une source d'ions 1, un spectromètre de masse 2 et un système de détection des ions 7. Le spectromètre de masse 2 est couplé en entrée à la source d'ions 1 pour recevoir les espèces ionisées issues de la source. Le spectromètre de masse 2 est couplé en sortie système de détection des ions 7, qui détecte les ions séparés par l'analyseur du spectromètre de masse en fonction de leur ratio masse sur charge. De plus, le spectromètre de masse 2 est couplé à une source 6 de rayonnement électromagnétique (laser, lampe ultra-violette, rayonnement synchrotron) ou à un faisceau d'électrons (canon à électron). Dans l'exemple de la figure 4, le spectromètre de masse 2 est de type triple quadrupole et comprend un premier quadrupole Q1, un deuxième quadrupole Q2 et un troisième quadrupole Q3. Le premier quadrupole Q1 permet de sélectionner un domaine de rapport m/z centré sur le rapport m/z de l'ion précurseur d'intérêt. Le deuxième quadrupole Q2 est couplé à une source 6 de rayonnement électromagnétique (laser, lampe UV, rayonnement synchrotron) ou à un faisceau d'électrons. Le deuxième quadrupole Q2, sert de cellule d'activation et comprend une région d'activation 4 du précurseur par interaction avec le faisceau de photons ou d'électrons de manière à induire une ionisation ou un détachement d'électron de l'ion précurseur. Le troisième quadrupole Q3 permet de filtrer les ions ayant subi une transition de charge d'intérêt en fonction de leur ratio masse sur charge (*m/z'*).

Le spectromètre de masse est ainsi configuré pour extraire, en direction du système de détection, un ion-produit ayant subi une transition de charge spécifique sans fragmentation pour permettre la détection de cet ion-produit spécifique.

Le système de détection 7 est adapté pour permettre la détection et la mesure de la ou des différents ion-produits ayant subi une ou respectivement plusieurs transition de charge d'intérêt.

Le système de détection 7 fournit, après un traitement du signal adapté, un spectre de masse. Le spectre de masse ainsi obtenu permet de fournir de nouvelles informations sur l'espèce moléculaire d'origine.

Avantageusement, le dispositif de spectrométrie de masse comprend des moyens de détection d'un ion précurseur ayant un ratio masse sur charge *(m/z)* prédéterminé et des moyens de détection d'un ion-produit ayant un ratio masse sur charge (*m/z*) tel que ledit ion-produit a la même masse m que ledit ion précurseur et un nombre de charge électrique *z'* tel que *z'* est un nombre entier non nul choisi parmi *z+1, z+2, z+3, z-1, z-2, z-3.*

Sur la figure 5 on a représenté un autre exemple de dispositif basé sur l'utilisation d'un piège à ion couplé à une source de rayonnement électromagnétique. Le dispositif de la figure 5 comprend une source d'ions 1, un capillaire de transfert des ions 11, un système optique ionique 21, un piège à ions quadrupolaire linéaire 12, un système de détection des ions 7, une lampe VUV, par exemple une lampe à décharge dans le néon produisant des photons de 16 eV environ, ou une lampe à décharge dans l'hélium produisant des photons de 21,22 eV environ, un laser 6 ou le rayonnement synchrotron et en option un obturateur 16 du faisceau de lumière 26 émis par le laser 6.

La source d'ions 1 génère au moins un ion précurseur en phase gazeuse à partir d'un analyte. Le capillaire de transfert des ions 11 permet de transférer les espèces ionisées en phase gazeuse depuis la source d'ions vers le piège à ions 12. Le système optique ionique 21 focalise les ions dans une zone du champ électromagnétique du piège à ions quadrupolaire linéaire 12 où l'ion précurseur se trouve piégée.

L'obturateur optionnel 16 étant ouvert, le faisceau laser 36 émis par le laser 6, la lampe à décharge ou le rayonnement synchrotron peut ainsi interagir dans le piège à ions 12 avec l'ion précurseur.

Typiquement, dans le cas d'ions précurseurs négatifs, un faisceau de photon d'énergie de 4 eV ou plus peut conduire au détachement d'électrons [Antoine R and Dugourd P 2011 Visible and ultraviolet spectroscopy of gas phase protein ions Phys. Chem. 13, 16494]. Un faisceau d'électrons d'énergie supérieure à 10 eV peut conduire au détachement d'électron [Zubarev R A 2003 Reactions of polypeptide ions with électrons in the gas phase. Mass spectrometry Reviews 22 57-77]. Typiquement, dans le cas d'ions positifs, des photons d'énergie supérieure à 10 eV peuvent conduire à la photoionisation selon l'état de charge du précurseur [Giuliani A, Milosavljevic A R, Hinsen K, Canon F, Nicolas C, Réfrégiers M and Nahon L 2012 Structure and Charge-State Dependence of the Gas-Phase Ionization Energy of Proteins Angewandte Chemie International Edition 51 9552-6]. Typiquement, un faisceau d'électrons d'énergie supérieure à 10 eV environ peut conduire à l'ionisation du précurseurs selon l'état de charge du précurseur [Fung Y M E, Adams C M and Zubarev R A 2009 Electron Ionization Dissociation of Singly and Multiply Charged Peptides J. Am. Chem. Soc 131 9977-85 ; Zubarev R A and Yang H 2010 Multiple Soft Ionization of Gas-Phase Proteins and Swift Backbone Dissociation in Collisions with ≤99 eV Electrons Angewandte Chemie International Edition 49, 1439-1441].

L'homme du métier adaptera le flux de photons de la source de photons et/ou sa puissance et sa longueur d'onde en fonction de l'énergie nécessaire pour une ionisation, un détachement d'électron selon certaines propriétés physico-chimique de l'ion cible, notamment la section efficace de photo-absorption de l'ion cible. L'homme de métier adaptera l'énergie cinétique des électrons en fonction de l'énergie nécessaire à apporter au système pour produire un détachement d'électron ou une ionisation selon certaines propriétés physico-chimique de l'ion précurseur cible.

Le piège à ions est configuré pour extraire des ion-produits correspondant à une transition de charge particulière et n'ayant pas subi de fragmentation. Connaissant le ratio masse sur charge (*m/z*) de l'ion précurseur, le système de détection 7 détecte spécifiquement les espèces ionisées ainsi activées, ou ion-produits, ayant un rapport masse sur charge égal à (*m/z*) tel que (*m/z*) de l'ion-produit est égal à (*m/(z-1))*, (*m/(z-2))*, (*m/(z-3))* ou (*m/(z+1))*, (*m/(z+2))*, (*m/(z+3))*.

Le système de détection d'ions 7 est configuré pour mesurer les ions ayant un ratio masse sur charge déterminé ou situé dans une gamme comprise entre un ration minimum et un ratio maximum. Il est ainsi possible de sélectionner les ion-produits ayant un ratio masse sur charge égal à *m/(z-1)* et/ou à *m/(z-2)* bien déterminé.

Pour stopper l'activation des ions, il suffit de fermer l'obturateur 16, d'arrêter le canon à électron ou bien de couper tout faisceau de particules interagissant avec les ions d'intérêt. On peut ainsi obtenir un spectre de masse des espèces moléculaires non activées et un autre spectre de masse des espèces moléculaires activées.

De préférence, l'analyseur de masse situé après ou autour de la région d'activation des ions et/ou le système de détection sont configurés pour filtrer les autres produits de l'activation par impact d'un faisceau de photons ou d'électrons avec les espèces moléculaires cible.

On note que l'interaction de l'analyte avec un faisceau de photons ou d'électrons peut générer simultanément des ions fragments de masse inférieure et ayant par conséquent un ratio masse sur charge différent de celui des ion-produits issus des réactions d'ionisation ou d'arrachement d'électron qui se produisent avec conservation de la masse de l'ion précurseur. De façon avantageuse, ces ions fragments sont filtrés par l'analyseur de masse situé entre la cellule d'activation et le système de détection.

Le procédé de l'invention peut être aisément implémenté sur des dispositifs de spectrométrie de masse existant.

En particulier, le dispositif et le procédé exposés ici sont compatibles avec tout arrangement instrumental dans lequel les étapes b) d'activation et c) de séparation d'ion-produit sont réalisées. On cite en particulier à titre d'exemple non-limitatif : un spectromètre quadrupôle - temps de vol comprenant une région d'activation, un spectromètre quadrupôle - piège à ion, un spectromètre de type triple quandrupôle, un spectromètre à temps de vol- temps de vol (Tof-Tof) ou encore un spectromètre hybride-orbitrap.

L'invention s'applique avantageusement au dosage, par spectrométrie de masse, de n'importe quelle espèce moléculaires ionisée en mélange complexe qui est impossible ou difficile à fragmenter, par les techniques habituelles d'activation, mais pour laquelle on puisse produire une ou plusieurs transitions de charge par interaction avec un faisceau d'espèces neutres, d'ions, d'électrons ou de photons.

Une application possible de la méthode concerne l'identification et le dosage de protéines entières. À titre d'exemple, nous avons comparé la méthode SRM et MRM classique avec la méthode proposée ici d'activation par transition de charge sans fragmentation de l'ion précurseur en mélange complexe.

Nous avons pris un extrait total de protéine de la bactérie E. Coli dans lequel nous avons ajouté de l'albumine de sérum bovin (BSA). Cette protéine ne peut pas être présente dans le lysat bactérien, les interférences potentiellement observées sont donc fortuites et proviennent de protéines bactériennes.

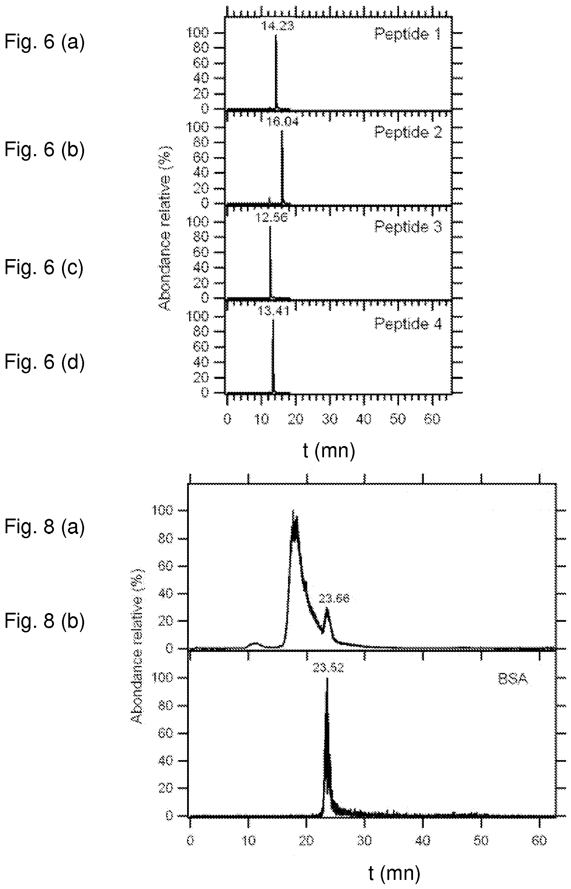

Dans une première expérience, nous avons appliqué le protocole SRM classique pour le dosage de la BSA tel que décrit dans la littérature. Un digestat standard de BSA (Protéabio) a été analysé par couplage chromatographie liquide - spectrométrie de masse. Pour l'approche SRM, ont été sélectionnés les quatre peptides les plus abondants, dont les rapports masse sur charge (m/z) sont respectivement les suivants : 582.55 (peptide 1), 740.61 (peptide 2), 927.5 (peptide 3) et 941.33 (peptide 4). Deux transitions ont été choisies pour chaque peptide avec une largeur de sélection et de détection de 1 Da parmi les fragmentations les plus intenses obtenues par dissociation induite par collision (CID) durant 30 ms et 35% d'énergie d'activation ; ces dissociations conduisant à des pics dans le spectre de masse respectivement : 573.06-574.06 et 950.86-951.86 (peptide 1), 812.96-813.96 et 1017.02-1018.02 (peptide 2), 892-893 et 909.92-910.92 (peptide 3), 931.69-932.69 et 1637.13-1638.13 (peptide 4). Les figures 6(a), 6(b), 6(c), 6(d) représentent les chromatogrammes d'ions extraits correspondants respectivement aux peptides 1, 2, 3 et 4 obtenus en fonction du temps de rétention sur la colonne de chromatographie. On mesure le temps de rétention qui correspond au temps que met un produit à être élué de la colonne de chromatographie. Les temps de rétention obtenus pour chaque peptide sont indiqués sur la figure 6, pour une colonne C18 (Gemini de chez Phenomenex, $3\mu$m, 110 Å, 100x2 mm) dans un gradient acétonitrile-eau de 60 minutes à 200 $\mu$L/min pour 150 pmol injectées.

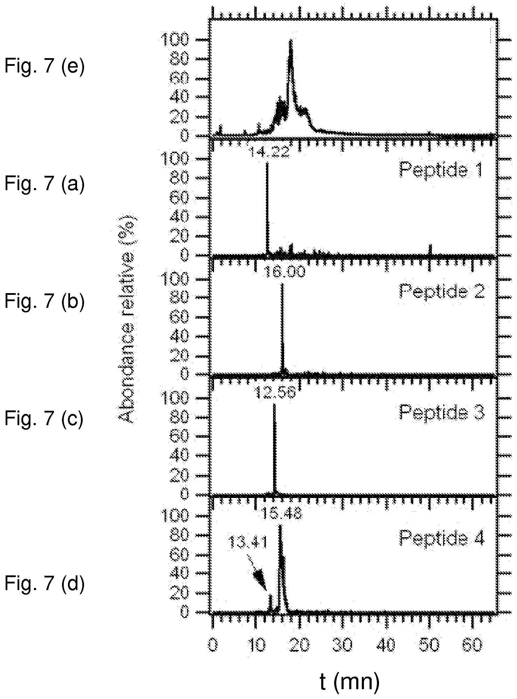

Dans une deuxième expérience, le lysat bactérien a été digéré à la trypsine et on a ajouté 5 pmol du digestat standard de BSA. La procédure classique de SRM a été appliquée pour la détection des peptides de BSA dans les mêmes conditions chromatographiques que sur la figure 6, et les résultats sont présentés à la figure 7.

Sur la figure 7(e), on a représenté le courant ionique total issu de l'ionisation de l'ensemble des peptides présents dans le mélange, en fonction du temps de rétention. Sur les figures 7(a), 7(b), 7(c), les peptides 1, 2 et 3 sont retrouvés aux mêmes temps de rétentions, mais pas le peptide 4. Pour ce dernier, sur la figure 7(d), une plusieurs autres espèces viennent parasiter la mesure et donc fausser la détection.

Dans une troisième expérience, l'analyse par SRM avec transition de charge sans fragmentation a été appliquée à un standard de BSA entière mélangé à l'extrait total brut bactérien. Le spectre électrospray de la BSA contient de très nombreux états de charge, et l'état de charge +48 donnant un m/z 1384.83 a été choisi. Pour l'analyse, on a sélectionné les trois transitions de charge ayant respectivement un ratio masse sur charge (avec une largeur de 5 Da) : 1301.08-1306.08 (état de charge +49), 1327.08-1332.08 (état de charge +50) et 1354.17-1359.17 (état de charge +51). Ces trois transitions de charge sont produites par irradiation d'un faisceau de photons à 21.22 eV. La figure 8 représente le chromatogramme de l'extrait bactérien auquel 18 pmol de BSA a été ajouté. La figure 8(a) représente la trace du courant ionique total due à l'ionisation de toutes les protéines présentes dans le milieu. La BSA est éluée à 23.52 minutes une séparation sur colonne C18 (Gemini de chez Phenomenex, $3\mu m$, 110 Å, 100x2 mm) dans un gradient acétonitrile-eau de 60 minutes à 200 $\mu$L/min. Sur la figure 8(b), on remarque que le chromatogramme de la BSA ne souffre d'aucune interférence. L'aire du pic chromatographique peut être aisément mesurée et reliée à la quantité de protéine ajoutée au milieu.

De cette comparaison entre la méthode SRM (ou MRM) classique et la méthode SRM (ou MRM) par transition de charge sans fragmentation, il ressort les avantages suivants :

- la méthode par transition de charge ne nécessite pas de digestion enzymatique de l'échantillon. Elle est donc plus universelle et peut s'appliquer à toutes les protéines, notamment celles qui résistent aux digestions (protéines riches en proline, protéines membranaires....) ;
- la relation entre la surface des pics d'élution et la quantité de matière injectée est directe et ne nécessite pas de combiner plusieurs traces chromatographiques ;
- la méthode par transition de charge est spécifique d'une espèce ionisée d'intérêt et ne subit pas d'interférence causée par des protéines de la matrice.
- Connaissant le ratio masse sur charge de l'ion précurseur, il est possible de calculer a priori les transitions de charges que donnera ledit ion précurseur.

## Revendications

1. Procédé de mesure par spectrométrie de masse d'espèces moléculaires dans un mélange complexe, le procédé comprenant les étapes suivantes :

   a. ionisation d'un analyte en mélange complexe pour former un ion précurseur (A, D) d'une espèce ionisée d'intérêt et au moins un ion interférent provenant du mélange complexe, l'ion précurseur (A, D) de ladite espèce ionisée d'intérêt et ledit au moins un ion interférent ayant un même ratio masse sur charge $m/z$, où m représente la masse et z le nombre de charge électrique de l'ion précurseur (A, D) de ladite espèce ionisée d'intérêt ;
   b. activation de l'ion précurseur (A, D) de ladite espèce ionisée d'intérêt et du au moins un ion interférent de même ratio masse sur charge, par interaction avec un faisceau d'espèces neutres, d'ions, d'électrons ou de photons, ayant une énergie prédéterminée en fonction de l'énergie d'ionisation de l'ion précurseur (A, D) de ladite espèce ionisée d'intérêt ou de l'énergie nécessaire au détachement d'électron de l'ion précurseur (A, D) de ladite espèce ionisée d'intérêt, ladite activation étant adaptée pour produire un ion-produit (B, C, E, F), par transition de charge sans fragmentation de l'ion précurseur (A, D) de ladite espèce ionisée d'intérêt, l'ion-produit (B, C, E, F) ayant la même masse m que l'ion précurseur (A, D) de ladite espèce ionisée d'intérêt et une charge électrique $z'$ telle que $z'$ est un nombre entier non nul différent de $z$, $z'$ étant de préférence choisi parmi $z+1$, $z+2$, $z+3$, $z-1$, $z-2$, $z-3$, de manière à ce que l'ion-produit (B, C, E, F) de ladite espèce ionisée d'intérêt ait un ratio masse sur charge $m/z'$ différent du ratio masse sur charge du ou des ions-produits issus par activation du au moins un ion interférent;
   c. séparation de l'ion-produit (B, C, E, F) de l'espèce ionisée d'intérêt ayant ledit ratio masse sur charge $m/z'$ prédéterminé spécifique de l'espèce ionisée d'intérêt ;
   d. détection sélective de l'ion-produit (B, C, F, E) de l'espèce ionisée d'intérêt ayant ledit ratio masse sur charge $m/z'$ prédéterminé spécifique de l'espèce ionisée d'intérêt.

2. Procédé de mesure selon la revendication 1 comportant une étape supplémentaire e) suite à l'étape d), l'étape e) comprenant une mesure de l'abondance de l'ion-produit (B, C, E, F) ayant ledit ratio masse sur charge $m/z'$ prédéterminé spécifique de l'espèce ionisée d'intérêt.

3. Procédé de mesure selon la revendication 1 ou 2, dans lequel ledit ion précurseur (D) de l'espèce ionisée d'intérêt a une polarité positive et en ce que l'étape b) d'activation comprend une étape d'ionisation sans fragmentation de manière à produire un ion-produit (E, F) ayant la même masse $m$ que ledit ion précurseur (D) de l'espèce ionisée d'intérêt et une polarité positive telle que le nombre de charge électrique $z'$ de l'ion-produit (E, F) est de préférence égal à $z+1$ par simple ionisation ou respectivement à $z+2$ par double ionisation.

**4.** Procédé de mesure selon la revendication 1 ou 2, dans lequel ledit ion précurseur (A) de l'espèce ionisée d'intérêt a une polarité négative et en ce que l'étape b) d'activation comprend une étape de détachement d'électron(s) sans fragmentation de manière à produire un ion-produit (B, C) ayant la même masse $m$ que ledit ion précurseur (A) de l'espèce ionisée d'intérêt et une polarité négative telle que le nombre de charge électrique $z'$ de l'ion-produit est de préférence égal à $z-1$ par simple détachement d'électron ou respectivement à $z-2$ par double détachement d'électron.

**5.** Procédé de mesure selon la revendication 1 ou 2, dans lequel ledit ion précurseur (D) de l'espèce ionisée d'intérêt a une polarité positive et en ce que l'étape b) d'activation comprend une étape de capture d'électron ou de transfert d'électron sans fragmentation de manière à produire un ion-produit (E, F) ayant la même masse $m$ que ledit ion précurseur (D) de l'espèce ionisée d'intérêt et une polarité positive telle que le nombre de charge électrique $z'$ de l'ion-produit (E, F) est de préférence égal à $z-1$ par simple capture ou transfert d'électron ou respectivement à $z-2$ par double capture ou transfert d'électron.

**6.** Procédé de mesure selon la revendication 1 ou 2, dans lequel ledit ion précurseur (A) de l'espèce ionisée d'intérêt a une polarité négative et en ce que l'étape b) d'activation comprend une étape de transfert d'électron sans fragmentation de manière à produire un ion-produit (B, C) ayant la même masse $m$ que ledit ion précurseur (A) de l'espèce ionisée d'intérêt et une polarité négative telle que le nombre de charge électrique $z'$ de l'ion-produit est de préférence égal à $z-1$ par simple transfert d'électron ou respectivement à $z-2$ par double transfert d'électron.

**7.** Procédé de mesure selon l'une des revendications précédentes comprenant en outre les étapes suivantes :

- une étape a1) suite à l'étape a) d'ionisation, l'étape a1) comprenant la détection et l'enregistrement d'un spectre de masse de référence ;
- une étape c1) de détection et d'enregistrement d'un spectre de masse tandem relatif à un ion précurseur de référence de ratio $(m/z)_{réf}$ donnant au moins un ion-produit (B, C, E, F) de ratio masse sur charge $(m/z')$ ; et
- une étape d1) de mesure de l'abondance de l'ion-produit (B, C, E, F) de l'espèce ionisée d'intérêt.

**8.** Procédé de mesure selon la revendication précédente dans lequel l'étape b) d'activation est réalisée par interaction d'un ion précurseur (A, D) avec un faisceau de photons issu d'un laser (6), d'une lampe à décharge ou d'une source de rayonnement synchrotron ou d'un faisceau d'électrons.

**9.** Procédé de mesure selon la revendication 7 dans lequel l'étape b) d'activation est réalisée par interaction d'un ion précurseur (A, D) avec un faisceau de photons ayant une énergie comprise entre 3 eV et 40 eV.

**10.** Procédé de mesure selon la revendication 7 dans lequel l'étape b) d'activation est réalisée par interaction d'un ion précurseur (A, D) avec un faisceau d'électrons ayant une énergie cinétique comprise entre 0 et 100 eV.

**11.** Procédé de mesure selon la revendication 7 dans lequel l'étape b) d'activation est réalisée par interaction d'un ion précurseur (A, D) avec un faisceau (26) d'ions énergétiques ayant une énergie comprise entre 0.1 et 10 keV.

**12.** Procédé de mesure selon l'une des revendications précédentes dans lequel les étapes b) d'activation et c) de séparation d'un ion-produit sont réalisées dans un piège à ions (12).

**13.** Application du procédé de mesure par spectrométrie de masse d'espèces moléculaires en mélange complexe selon l'une des revendications 1 à 12 au dosage de protéines, de bio-polymères, de polymères, de macromolécules, de virus, de nanoparticules, de micro-organismes d'assemblages supramoléculaires ou d'assemblages de bio-polymères.

**Patentansprüche**

**1.** Verfahren zum Messen molekularer Spezies in einer komplexen Mischung mittels Massenspektroskopie, wobei das Verfahren die folgenden Schritte aufweist:

a. Ionisieren eines Analyten in komplexer Mischung zum Bilden eines Vorläuferions (A, D) einer betreffenden ionisierten Spezies und wenigstens eines interferierenden Ions, das aus der komplexen Mischung stammt, wobei das Vorläuferion (A, D) der betreffenden ionisierten Spezies und das besagte wenigstens eine interferierende Ion das gleiche Masse/Ladung-Verhältnis m/z aufweisen, wobei m die Masse und z die Anzahl elektrischer Ladungen des Vorläuferions (A, D) der betreffenden ionisierten Spezies ist;

b. Aktivieren des Vorläuferions (A, D) der betreffenden ionisierten Spezies und des wenigstens einen interferierenden Ions gleichen Masse/Ladung-Verhältnisses durch Zusammenwirken mit einem Strahl neutraler Spezien, Ionen, Elektronen oder Photonen, die eine in Abhängigkeit von der Ionisationsenergie des Vorläuferions (A, D) der betreffenden ionisierten Spezies oder der zum Abtrennen des Vorläuferions (A, D) von der betreffenden ionisierten Spezies notwendigen Energie vorbestimmte Energie aufweisen, wobei die Aktivierung zur Erzeugung eines Produktion (B, C, E, F) durch Ladungsübertragung ohne Fragmentierung des Vorläuferions (A, D) der betreffenden ionisierten Spezies angepaßt ist, wobei das Produktion (B, C, E, F) die gleiche Masse m wie das Vorläuferion (A, D) der betreffenden ionisierten Spezies und eine elektrische Ladung z' derart aufweist, daß z' eine von Null verschiedene ganze Zahl ist, wobei z' vorzugsweise aus z+1, z+2, z+3, z-1, z-2, z-3 ausgewählt ist, so daß das Produktion (B, C, E, F) der betreffenden ionisierten Spezies ein Masse/Ladung-Verhältnis m/z' aufweist, das sich vom Masse/Ladung-Verhältnis des aus der Aktivierung des wenigstens einen interferierenden Ions hervorgegangenen Produktions bzw. der Produktionen unterscheidet;

c. Trennen des Produktions (B, C, E, F) der betreffenden ionisierten Spezies, das das spezifische vorbestimmte Masse/Ladung-Verhältnis m/z' der betreffenden ionisierten Spezies aufweist;

d. selektives Erkennen des Produktions (B, C, E, F) der betreffenden ionisierten Spezies, das das spezifische vorbestimmte Masse/Ladung-Verhältnis m/z' der betreffenden ionisierten Spezies aufweist.

2. Meßverfahren gemäß Anspruch 1, das einen zusätzlichen Schritt e) im Anschluß an den Schritt d) aufweist, wobei der Schritt e) das Messen des Aufkommens an Produktionen (B, C, E, F) mit dem spezifischen vorbestimmten Masse/Ladung-Verhältnis m/z' der betreffenden ionisierten Spezies beinhaltet.

3. Meßverfahren gemäß Anspruch 1 oder 2, bei dem das Vorläuferion (D) der betreffenden ionisierten Spezies eine positive Polarität aufweist und bei dem der Schritt b) des Aktivierens einen Ionisationsschritt ohne Fragmentierung aufweist, um ein Produktion (E, F) zu erzeugen, das die gleiche Masse m wie das Vorläuferion (D) der betreffenden ionisierten Spezies und eine negative Polarität derart aufweist, daß die Zahl z' der elektrischen Ladungen des Produktions (E, F) vorzugsweise gleich z+1 durch einfache Ionisation oder gleich z+2 durch doppelte Ionisation ist.

4. Meßverfahren gemäß Anspruch 1 oder 2, bei dem das Vorläuferion (A) der betreffenden ionisierten Spezies eine negative Polarität aufweist und bei dem der Schritt b) des Aktivierens einen Schritt des Abtrennens von Elektronen ohne Fragmentierung aufweist, um ein Produktion (B, C) zu erzeugen, das die gleiche Masse m wie das Vorläuferion (A) der betreffenden ionisierte Spezies und eine negative Polarität derart aufweist, daß die Zahl z' der elektrischen Ladungen des Produktions vorzugsweise gleich z-1 durch einfaches Elektronenabtrennen oder gleich z-2 durch doppeltes Elektronenabtrennen ist.

5. Meßverfahren gemäß Anspruch 1 oder 2, bei dem das Vorläuferion (D) der betreffenden ionisierten Spezies eine positive Polarität aufweist und bei dem der Schritt b) des Aktivierens einen Schritt des Einfangens von Elektronen oder des Übertragens von Elektronen ohne Fragmentierung aufweist, um ein Produktion (E, F) zu erzeugen, das die gleiche Masse m wie das Vorläuferion (D) der betreffenden ionisierten Spezies und eine positive Polarität derart aufweist, daß die Zahl z' der elektrischen Ladungen des Produktions (E, F) vorzugsweise gleich z-1 durch einfaches Einfangen oder Übertragen von Elektronen oder gleich z-2 durch doppeltes Einfangen oder Übertragen von Elektronen ist.

6. Meßverfahren gemäß Anspruch 1 oder 2, bei dem das Vorläuferion (A) der betreffenden ionisierten Spezies eine negative Polarität aufweist und bei dem der Schritt b) des Aktivierens einen Schritt des Übertragens von Elektronen ohne Fragmentierung aufweist, um ein Produktion (B, C) zu erzeugen, das die gleiche Masse m wie das Vorläuferion (A) der betreffenden ionisierten Spezies und eine negative Polarität derart aufweist, daß die Zahl z' der elektrischen Ladungen des Produktions vorzugsweise gleich z-1 durch einfaches Übertragen von Elektronen oder gleich z-2 durch doppeltes Übertragen von Elektronen ist.

7. Meßverfahren gemäß einem der vorangehenden Ansprüche, das außerdem die folgenden Schritte aufweist:

- einen Schritt a1) im Anschluß an den Schritt a) des Ionisierens, wobei der Schritt a1) das Erfassen und Registrieren eines Referenzmassenspektrums aufweist,

- einen Schritt c1) des Erfassens und Registrierens eines Tandemmassenspektrums bezüglich eines Referenzvorläuferions mit einem Verhältnis $(m/z)_{ref}$, das wenigstens ein Produktion (B, C, E, F) mit dem Masse/Ladung-Verhältnis (m/z') ergibt, und

- einen Schritt d1) des Messens des Aufkommens an Produktionen (B, C, E, F) der betreffenden ionisierten Spezies.

8. Meßverfahren gemäß dem vorangehenden Anspruch, bei dem der Schritt b) des Aktivierens durch Zusammenwirken eines Vorläuferions (A, D) mit einem von einem Laser (6), einer Entladungslampe oder einer Synchrotron-Strahlenquelle oder einem Elektronenstrahl ausgehenden Photonenstrahl ausgeführt wird.

9. Meßverfahren gemäß Anspruch 7, bei dem der Schritt b) des Aktivierens durch Zusammenwirken eines Vorläuferions (A, D) mit einem Photonenstrahl ausgeführt wird, der eine Energie zwischen 3 eV und 40 eV aufweist.

10. Meßverfahren gemäß Anspruch 7, bei dem der Schritt b) des Aktivierens durch Zusammenwirken eines Vorläuferions (A, D) mit einem Elektronenstrahl ausgeführt wird, der eine kinetische Energie zwischen 0 und 100 eV aufweist.

11. Meßverfahren gemäß Anspruch 7, bei dem der Schritt b) des Aktivierens durch Zusammenwirken eines Vorläuferions (A, D) mit einem Strahl (26) aus energiereichen Ionen ausgeführt wird, der eine Energie zwischen 0,1 und 10 keV aufweist.

12. Meßverfahren gemäß einem der vorangehenden Ansprüche, bei dem die Schritte b) des Aktivierens und c) des Trennens eines Produktions in einer Ionenfalle (12) ausgeführt werden.

13. Anwendung des Verfahrens zum Messen molekularer Spezies in einer komplexen Mischung mittels Massenspektroskopie gemäß einem der Ansprüche 1 bis 12 bei der Dosierung von Proteinen, von Biopolymeren, von Polymeren, von Makromolekülen, von Viren, von Nanopartikeln, von Mikroorganismen supramolekularer Zusammensetzungen oder biopolymerer Zusammensetzungen.

## Claims

1. A method for mass spectrometry measurement of molecular species in a complex mixture, the method comprising the following steps:

   a. ionising an analyte in complex mixture to form a precursor ion (A, D) of an ionised species of interest and at least one interfering ion coming from the complex mixture, the precursor ion (A, D) of said ionised species of interest and said at least one interfering ion having a same mass-to-charge ratio $m/z$, where m represents the mass and z the electric charge number of the precursor ion (A, D) of said ionised species of interest;
   b. activating the precursor ion (A, D) of said ionised species of interest and of the at least one interfering ion of same mass-to-charge ratio, by interaction with a beam of neutral species, ions, electrons or photons, having a predetermined energy as a function of the energy of ionisation of the precursor ion (A, D) of said ionised species of interest or of the energy required for the detachment of electron from the precursor ion (A, D) of said ionised species of interest, said activation being adapted to produce a product-ion (B, C, E, F), by charge transition with no fragmentation of the precursor ion (A, D) of said ionised species of interest, the product-ion (B, C, E, F) having the same mass $m$ as the precursor ion (A, D) of said ionised species of interest and an electric charge $z'$ such that $z'$ is a non-zero integer number different from $z$, $z'$ being preferably chosen among $z+1$, $z+2$, $z+3$, $z-1$, $z-2$, $z-3$, so that the product-ion (B, C, E, F) of said ionised species of interest has a mass-to-charge ratio $m/z'$ different from the mass-to-charge ratio of the product-ion(s) stemming from the activation of the at least one interfering ion;
   c. separating the product-ion (B, C, E, F) of the ionised species of interest having said predetermined mass-to-charge ratio m/z' specific to the ionised species of interest;
   d. selectively detecting the product-ion (B, C, F, E) of the ionised species of interest having said predetermined mass-to-charge ratio $m/z'$ specific to the ionised species of interest.

2. The measurement method according to claim 1, including an additional step e) after the step d), the step e) comprising a measurement of the abundance of the product-ion (B, C, E, F) having said predetermined mass-to-charge ratio m/z' specific to the ionised species of interest.

3. The measurement method according to claim 1 or 2, wherein said precursor ion (D) of the ionised species of interest has a positive polarity and the step b) of activation comprises a step of ionisation with no fragmentation so as to produce a product-ion (E, F) having the same mass $m$ as said precursor ion (D) of the ionised species of interest and a positive polarity such that the electric charge number $z'$ of the product-ion (E, F) is preferably equal to $z+1$ by simple ionisation, or respectively to *z+2* by double ionisation.

4. The measurement method according to claim 1 or 2, wherein said precursor ion (A) of the ionised species of interest has a negative polarity and the step b) of activation comprises a step of detachment of electron(s) with no fragmentation so as to produce a product-ion (B, C) having the same mass *m* as said precursor ion (A) of the ionised species of interest and a negative polarity such that the electric charge number *z'* of the product-ion is preferably equal to *z-1* by simple electron detachment, or respectively to *z-2* by double electron detachment.

5. The measurement method according to claim 1 or 2, wherein said precursor ion (D) of the ionised species of interest has a positive polarity and the step b) of activation comprises a step of electron capture or of electron transfer with no fragmentation so as to produce a product-ion (E, F) having the same mass m as said precursor ion (D) of the ionised species of interest and a positive polarity such that the electric charge number *z'* of the product-ion (E, F) is preferably equal to *z-1* by simple electron capture or transfer, or respectively to *z-2* by double electron capture or transfer.

6. The measurement method according to claim 1 or 2, wherein said precursor ion (A) of the ionised species of interest has a negative polarity and the step b) of activation comprises a step of electron transfer with no fragmentation so as to produce a product-ion (B, C) having the same mass *m* as said precursor ion (A) of the ionised species of interest and a negative polarity such that the electric charge number z' of the product-ion is preferably equal to z-1 by simple electron transfer, or respectively to *z-2* by double electron transfer.

7. The measurement method according to one of the preceding claims, further comprising the following steps:

   - a step a1) after the step a) of ionisation, the step a1) comprising the detection and the recording of a reference mass spectrum;
   - a step c1) of detection and recording of a tandem mass spectrum relating to a reference precursor ion of ratio $(m/z)_{ref}$ giving at least one product-ion (B, C, E, F) of mass-to-charge ratio $(m/z')$; and
   - a step d1) of measurement of the abundance of the product-ion (B, C, E, F) of the ionised species of interest.

8. The measurement method according to the preceding claim, wherein the step b) of activation is performed by interaction of a precursor ion (A, D) with a photon beam stemming from a laser (6), a discharge lamp or a synchrotron radiation source or an electron beam.

9. The measurement method according to claim 7, wherein the step b) of activation is performed by interaction of a precursor ion (A, D) with a photon beam having an energy comprised between 3 eV and 40 eV.

10. The measurement method according to claim 7, wherein the step b) of activation is performed by interaction of a precursor ion (A, D) with an electron beam having a kinetic energy comprised between 0 and 100 eV.

11. The measurement method according to claim 7, wherein the step b) of activation is performed by interaction of a precursor ion (A, D) with a beam (26) of energetic ions having an energy comprised between 0.1 and 10 keV.

12. The measurement method according to any one of the preceding claims, wherein the steps b) of activation and c) of separation of a product-ion are performed in an ion trap (12).

13. An application of the method for mass spectrometry measurement of molecular species in a complex mixture according to one of claims 1 to 12 to the dosage of proteins, biopolymers, polymers, macromolecules, virus, nanoparticles, micro-organisms, supramolecular assemblies or biopolymers assemblies.

## Fig. 4

| 1 | Q1<br>3 | Q2<br>4 | Q3<br>5 | 7 |

2

6

## Fig. 5

1    11    21    12    16    6

7

7

Fig. 6 (a)

Fig. 6 (b)

Fig. 6 (c)

Fig. 6 (d)

Fig. 8 (a)

Fig. 8 (b)

Fig. 7 (e)

Fig. 7 (a)

Fig. 7 (b)

Fig. 7 (c)

Fig. 7 (d)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2012066261 A1
- WO 2006064132 A1

**Littérature non-brevet citée dans la description**

- **P. PICOTTI ; R. AEBERSOLD.** Selected reaction monitoring-based proteomics : workflows, potential, pitfalls and future directions. *Nature Methods,* 2012, vol. 9, 555-66
- **A. KALLI ; K. HAKANSSON.** Electron capture dissociation of highly charged proteolytic peptides from Lys N, Lys C and Glu C digestion. *Mol. Biosyst.,* 2010, vol. 6, 1668-1681
- **LEMOINE J ; DUGOURD P ; SALVADOR A ; ANTOINE R ; BRETONNIERE Y.** *Brevet FR,* 2010, vol. 2967, 780
- **ANTOINE R ; DUGOURD P.** Visible and ultraviolet spectroscopy of gas phase protein ions. *Phys. Chem.,* 2011, vol. 13, 16494
- **GIULIANI A ; MILOSAVLJEVIC A R ; HINSEN K ; CANON F ; NICOLAS C ; RÉFRÉGIERS M ; NAHON L.** *Structure and Charge-State Dependence of the Gas-Phase Ionization Energy of Proteins Angewandte Chemie International Edition,* 2012, vol. 51, 9552-6
- **FUNG Y M E ; ADAMS C M ; ZUBAREV R A.** Electron Ionization Dissociation of Singly and Multiply Charged Peptides. *J. Am. Chem. Soc,* 2009, vol. 131, 9977-85
- **ZUBAREV R A ; YANG H.** Multiple Soft Ionization of Gas-Phase Proteins and Swift Backbone Dissociation in Collisions with ≤99 eV Electrons. *Angewandte Chemie International Edition,* 2010, vol. 49, 1439-1441